# EUROPÄISCHE PATENTSCHRIFT

(11) **EP 4 031 698 B1**
(45) Veröffentlichungstag und Bekanntmachung des Hinweises auf die Patenterteilung: **05.06.2024**
(21) Anmeldenummer: 20775815.2
(22) Anmeldetag: 15.09.2020
(51) Int. Cl.: D01G 31/00, B65H 63/06, D01H 13/22, G01N 33/36

(54) **OPTIMIERUNG EINES GARNHERSTELLUNGSPROZESSES BEZÜGLICH FREMDMATERIALIEN.**
OPTIMIZING A YARN PRODUCTION PROCESS WITH RESPECT TO FOREIGN MATERIALS
OPTIMISATION D'UN PROCESSUS DE PRODUCTION DE FIL PAR RAPPORT À DES MATIÈRES ÉTRANGÈRES

(30) Priorität: 17.09.2019 CH 11762019
(43) Veröffentlichungstag der Anmeldung: 27.07.2022
(73) Patentinhaber: Uster Technologies AG, 8610 Uster (CH)
(72) Erfinder: SCHNEIDER, Ulf, 8610 Uster (CH); SIEGENTHALER, Mario, 8535 Herdern (CH)
(74) Vertreter: Pliska, Pavel
(86) Internationale Anmeldenummer: PCT/CH2020/000012
(87) Internationale Veröffentlichungsnummer: WO 2021/051210

(56) Entgegenhaltungen:
- EP-A1- 1 123 995
- WO-A1-2007/010325
- WO-A1-2017/190259
- WO-A1-2019/173929
- DE-A1-102010 055 523
- DE-U1- 29 622 931

## Beschreibung

### FACHGEBIET

Die vorliegende Erfindung liegt auf dem Gebiet der Herstellung von Garn. Sie betrifft ein Verfahren und eine Vorrichtung zur Optimierung eines Garnherstellungsprozesses, in dem Fremdmaterialien in einem Textilfasergebilde überwacht werden, gemäss den unabhängigen Patentansprüchen.

### STAND DER TECHNIK

Fremdmaterialien im Garn stellen eines der grossen Probleme heutiger Spinnereien dar. Es handelt sich dabei um Materialien, die sich vom beabsichtigten Grundmaterial der Garnfasern, z. B. Baumwollfasern, unterscheiden. Sie können verschiedenen Ursprungs sein, wie z. B. Kunststoffverpackungen, Schnüre, menschliche oder tierische Haare etc. Fremdmaterialien führen zu Fadenbrüchen beim Spinnen und Weben, nehmen Farbstoff in anderer Weise an als das Grundmaterial und beeinflussen das Aussehen des textilen Endproduktes. Sie vermindern wesentlich den Wert des Endproduktes. Eine Übersicht über Gewebefehler, die durch Fremdmaterialien verursacht sind, und Empfehlungen zu ihrer Verminderung gibt Abs. 3.8 des USTER^{®} *NEWS BULLETIN NO. 47* "The origins of fabric defects - and ways to reduce them", Uster Technologies AG, März 2010.

Die WO-2006/079426 A1 offenbart ein Verfahren und eine Vorrichtung zum Ausscheiden von Fremdstoffen in Fasermaterial, insbesondere in Rohbaumwolle. Derartige Verfahren werden beispielsweise in der Putzerei eingesetzt, um die Rohbaumwolle für das Spinnen vorzubereiten. Das Fasermaterial wird in einer pneumatischen Fasertransportleitung nacheinander an einem Sensorsystem und an einer Ausscheidevorrichtung vorbeigeführt. Beim Erkennen von Fremdmaterialien durch das Sensorsystem werden diese mittels eines quer zur Fasertransportleitung gerichteten Druckluftimpulses durch eine Ausscheideöffnung in der Fasertransportleitung aus dieser ausgeschieden. Ein entsprechender Faserreiniger ist in der Broschüre "USTER^{®} *JOSSI VISION SHIELD 2-*The key to Total Contamination Control", Uster Technologies AG, Oktober 2015, beschrieben.

Weiter stromabwärts im textilen Herstellungsprozess können Fremdmaterialien auf Spinn- oder Spulmaschinen durch so genannte Garnreiniger aus dem Garn entfernt werden. Ein Garnreiniger beinhaltet einen Messkopf mit mindestens einem Sensor, der das bewegte Garn abtastet und dabei Garnfehler wie Fremdmaterialien oder Dick- und Dünnstellen detektiert. Das Ausgangssignal des Sensors wird laufend gemäss vorgegebenen Kriterien ausgewertet. Die US-6,244,030 B1 offenbart einen Garnreiniger, der nicht nur Fremdmaterialien detektiert, sondern auch verschiedene Arten von Fremdmaterialien voneinander unterscheidet. Der Sensor tastet das Garn optisch durch Auflicht ab. Es wird ein Klassierfeld oder eine Klassiermatrix zur Verfügung gestellt. Längs der horizontalen Achse des Klassierfeldes wird die Länge von Gamabschnitten und längs der vertikalen Achse wird die Reflektivität von Licht am Garn aufgetragen. Das Klassierfeld ist in 16 Klassen für helle Fremdmaterialien und 16 Klassen für dunkle Fremdmaterialien unterteilt. Garnabschnitte der gleichen Klasse werden gezählt. Ein entsprechendes Produkt ist in der Broschüre "USTER^{®} *QUANTUM 3* Application Handbook", Abs. 8.4, Uster Technologies AG, April 2011, beschrieben. Ein Garnreiniger, der das Garn mit mehreren verschiedenfarbigen Lichtkomponenten abtastet, ist aus der WO-2011/026249 A1 bekannt.

Die WO-2017/190259 A1 beschreibt ein Verfahren und eine Vorrichtung zur Überwachung von Verunreinigungen in einem Faserflockenstrom. In einer Ausführungsform überwacht eine erste Überwachungsvorrichtung Verunreinigungen in einem Faserflockenstrom, während eine zweite Überwachungsvorrichtung Verunreinigungen stromabwärts im textilen Herstellungsprozess überwacht. Die zweite Überwachungsvorrichtung kann ein Garnreiniger auf einer Spulmaschine sein. Eine Steuereinheit ist mit der ersten und der zweiten Überwachungsvorrichtung verbunden. Sie sammelt Daten von den beiden Überwachungsvorrichtungen, wertet sie statistisch aus und gibt daraus hergestellte Berichte an eine Bedienungsperson aus. In einem Regelkreis wird eine Grenze zur Entfernung der Verunreinigungen in der ersten Überwachungsvorrichtung abhängig von einen Überwachungsresultat der zweiten Überwachungsvorrichtung geändert.

In der nach dem Prioritätstag des vorliegenden Schutzrechts veröffentlichten WO-2019/173929 A1 wird ein Verfahren zur Optimierung eines Spinnprozesses bezüglich Fremdmaterialien beschrieben. An einer ersten Stelle des Spinnprozesses wird ein Strom von Faserflocken, die pneumatisch in einem Luftstrom gefördert werden, auf Fremdmaterialien überwacht. Aufgrund der Überwachung werden Fremdmaterialien gemäss einem Ausscheidungskriterium aus dem Strom von Faserflocken ausgeschieden. An einer zweiten Stelle des Spinnprozesses, die stromabwärts bezüglich der ersten Stelle liegt, wird Garn, das aus den Faserflocken gesponnen wurde und entlang seiner Längsrichtung gefordert wird, auf Fremdmaterialien überwacht. Aufgrund der Überwachung werden die im Garn detektierten Fremdmaterialien gemäss mindestens einer Eigenschaft der Fremdmaterialien in mindestens zwei Klassen klassiert. Ein Anteil der Fremdmaterialien in mindestens einer der mindestens zwei Klassen an einer Gesamtanzahl der im Garn detektierten Fremdmaterialien wird bestimmt. Das Ausscheidungskriterium wird in Abhängigkeit vom Anteil der Fremdmaterialien in der mindestens einen Klasse geändert. Eine Ausführungsform verwendet mehrere Teilbereiche des elektromagnetischen Spektrums.

Die US-6,452,157 B1 befasst sich mit einer Vorrichtung an einer Faserverarbeitungseinrichtung zum Erkennen und Verarbeiten von Verunreinigungen, Fremdstoffen und -fasern in textilem Fasermaterial. Die Vorrichtung hat mindestens zwei Lichtquellen, die das Fasermaterial abwechselnd mit unterschiedlicher Farbe beleuchten. Ausserdem ist ein Sensor vorgesehen, der die Farben des vom Fasermaterial reflektierten Lichtes empfängt. Bei einer sprunghaften Farbänderung des Fasermaterials von einer vorgegebenen Farbe entsteht ein elektrisches Signal. Um eine Anpassung der Leuchtfarben je nach Anwendungsfall, z. B. an unterschiedliche oder wechselnde Farbe des Fasermaterials, zu ermöglichen, wird eine Mehrfarben-Lichtquelle mit mehr als zwei Farben herangezogen. Die Art der Farben der Mehrfarben-Lichtquelle ist in Abhängigkeit von der Farbe des zu beleuchtenden Fasermaterials wählbar.

Die DE-296'22'931 U1 beschreibt eine Vorrichtung in einer Spinnereivorbereitungsanlage zum Erkennen und Auswerten von Fremdstoffen in bzw. aus Fasergut. Ein optisches Sensorsystem ist an eine Bildverarbeitungseinrichtung angeschlossen, die mit einer elektronischen Regel- und Steuereinrichtung in Verbindung steht. Zur Beleuchtung des Fasergutes können Lichtquellen mit verschiedenen Wellenlängen Anwendung finden. Nach einer Lernphase wird die Ausscheideempfindlichkeit für die Fremdstoffe festgelegt, wobei die Grenzen automatisch und/oder halbautomatisch variiert werden können. Messsignale der Bildverarbeitungseinrichtung werden mit gespeicherten Sollwerten verglichen. Fremdmaterial mit abweichender Charakteristik wird aus dem Fasergut entfernt.

Die DE-10'2010'055'523 A1 offenbart eine Vorrichtung in der Spinnereivorbereitung zum Erkennen von Fremdteilen aus Kunststoff in oder zwischen Faserflocken aus Baumwolle. Fasermaterial wird zum einen mit unpolarisiertem Licht mit einem Wellenlängenbereich beleuchtet und gleichzeitig mit polarisiertem Licht eines anderen Wellenlängenbereiches beleuchtet, derart, dass beide Lichtarten gemeinsam in einer Maschine an nahe beieinander liegenden Inspektionsstellen anwendbar sind. Dadurch soll die Erkennung auch solcher Kunststoffabfälle in Fasermaterialien ermöglicht werden, welche nicht mit polarisiertem Licht erkennbar sind (nicht transparent) und auch nicht mit UV-Licht erkennbar sind (nicht fluoreszierend),

Gemäss der EP-1' 123'995 A1 werden textile Fasern in einer Behandlungsanlage mittels eines Transportsystems nacheinander verschiedenen Behandlungsstationen zugeführt. Dabei werden die Fasern wenigstens gereinigt und homogenisiert. Mit einem Sensorsystem werden laufend wenigstens zwei unterschiedliche physikalische Messgrössen an den Fasern ermittelt. Das Sensorsystem kann Infrarotsensoren beinhalten, die mit zwei wenigstens charakteristischen Frequenzen arbeiten. Aus den ermittelten Messgrössen werden bestimmte Fasereigenschaften abgeleitet und Istwerte gebildet, die in einer Auswerteeinrichtung mit einem Sollwert für jede Fasereigenschaft verglichen werden. Beim Vorliegen von Abweichungen von einem Sollwert wird der Betriebszustand wenigstens einer Behandlungsstation und/oder des Transportsystems verändert. Damit soll der gesamte Behandlungsprozess optimiert werden.

Die WO-2007/010325 A1 beschreibt eine Vorrichtung zum Detektieren von Fremdstoffen in einem strangförmigen oder flockenförmigen Textilmaterial. Die Vorrichtung umfasst eine erste Lichtquelle zum Emittieren von sichtbarem Licht, eine zweite Lichtquelle zum Emittieren von Strahlung im infraroten Spektrum, einen für sichtbares Licht empfindlichen ersten Detektor und einen für Strahlung im nahen infraroten Spektrum empfindlichen zweiten Detektor. Die Lichtquellen und Detektoren sind angeordnet, um denselben Abschnitt des Garns zu beleuchten und zu betrachten. Ein Signalverarbeitungsabschnitt ist konfiguriert, um das Verhältnis und eine gewichtete Differenz der beiden Detektorsignale zu bestimmen und ein Signal auszugeben, welches das Vorhandensein einer Verunreinigung anzeigt, wenn die Differenz einen Schwellenwert überschreitet.

### DARSTELLUNG DER ERFINDUNG

Es ist eine Aufgabe der vorliegenden Erfindung, ein Verfahren und eine verbesserte Vorrichtung zur Optimierung eines Garnherstellungsprozesses, in dem Fremdmaterialien in einem Textilfasergebilde überwacht werden, anzugeben.

Diese und andere Aufgaben werden durch das erfindungsgemässe Verfahren und die erfindungsgemässe Vorrichtung gelöst, wie sie in den unabhängigen Patentansprüchen definiert sind. Vorteilhafte Ausführungsformen sind in den abhängigen Patentansprüchen angegeben.

Die Erfindung beruht auf der Idee, die Fremdmaterialien je nach ihrer Farbe in verschiedene Farbklassen zu klassieren. Wenn eine Stichprobe von klassierten Fremdmaterialien vorliegt, wird eine Häufigkeitsverteilung der Fremdmaterialien für die Farbklassen ermittelt. Bei einer Abweichung der Häufigkeitsverteilung von einer Referenz-Häufigkeitsverteilung wird eine Optimierungshandlung vorgenommen, z. B. ein Warnsignal ausgegeben.

Gemäss der Erfindung wird also erst nachträglich, bei Vorliegen einer statistisch relevanten Stichprobe, eine statistische Auswertung der klassierten Fremdmaterialien vorgenommen. Dadurch kann der Garnherstellungsprozess mittel- und langfristig optimiert werden. Die Erfindung zielt somit nicht auf eine sofortige Reaktion, wie z. B. eine einzelne Ausscheidung eines Fremdmaterials, ab.

Bei Kenntnis der Erfindung ist der Fachmann in der Lage, den erforderlichen Umfang der Stichprobe zu bestimmen und die Relevanz der Abweichung zu beurteilen. Der Stichprobenumfang und die Abweichungsrelevanz hängen teilweise von den jeweiligen Gegebenheiten ab. In vielen Fällen werden einfache, empirische Verfahren genügen. Die entsprechenden Grundlagen für eine theoretische Betrachtung sind z. B. im Buch «Statistische Methoden bei textilen Untersuchungen» von Graf, Henning und Wilrich, 2. Aufl., Springer-Verlag, 1974, dargelegt.

Das erfindungsgemässe Verfahren dient zur Optimierung eines Garnherstellungsprozesses, in dem Fremdmaterialien in einem Textilfasergebilde überwacht werden. Das Textilfasergebilde wird mit elektromagnetischer Strahlung aus mindestens zwei unterschiedlichen Teilbereichen des elektromagnetischen Spektrums angestrahlt. Die elektromagnetische Strahlung wechselwirkt mit den Fremdmaterialien. Fremdmaterialien werden aufgrund ihrer Wechselwirkung mit der elektromagnetischen Strahlung detektiert. Jedem der mindestens zwei unterschiedlichen Teilbereiche des elektromagnetischen Spektrums wird eine Farbklasse von Fremdmaterialien in Abhängigkeit von der Wechselwirkung der elektromagnetischen Strahlung in dem betreffenden Teilbereich des elektromagnetischen Spektrums mit den Fremdmaterialien zugeordnet. Die detektierten Fremdmaterialien werden je nach ihrer Wechselwirkung mit der elektromagnetischen Strahlung in dem betreffenden Teilbereich des elektromagnetischen Spektrums in den mindestens zwei Farbklassen automatisch klassiert. Bei Vorliegen einer Stichprobe mit einer Vielzahl von klassierten Fremdmaterialien wird automatisch eine Häufigkeitsverteilung der Fremdmaterialien für die Farbklassen ermittelt. Die ermittelte Häufigkeitsverteilung wird automatisch mit einer Referenz-Häufigkeitsverteilung verglichen. Bei einer signifikanten Abweichung der ermittelten Häufigkeitsverteilung von der Referenz-Häufigkeitsverteilung wird mindestens eine aus einer Menge von mehreren Optimierungshandlungen vorgenommen.

Die Menge von mehreren Optimierungshandlungen kann mindestens eines der folgenden Elemente beinhalten:
- ein Warnsignal wird ausgegeben;
- eine Empfehlung wird ausgegeben;
- die ermittelte Häufigkeitsverteilung wird ausgegeben.

In einer Ausführungsform wird für jede der mindestens zwei Farbklassen ein Ausscheidungskriterium definiert. Fremdmaterialien werden gemäss den mindestens zwei Ausscheidungskriterien aus dem Textilfasergebilde ausgeschieden. Die Häufigkeitsverteilung bezieht sich auf die aus dem Textilfasergebilde ausgeschiedenen und/oder die in dem Textilfasergebilde verbliebenen Fremdmaterialien. In dieser Ausführungsform kann die Menge von mehreren Optimierungshandlungen eines der folgenden Elemente beinhalten:
- das für die betreffende Farbklasse definierte Ausscheidungskriterium wird so geändert, dass bei unverändertem Textilfasergebilde in der betreffenden Farbklasse mehr Fremdmaterialien aus dem Textilfasergebilde ausgeschieden werden;
- das für die betreffende Farbklasse definierte Ausscheidungskriterium wird so geändert, dass bei unverändertem Textilfasergebilde in der betreffenden Farbklasse weniger Fremdmaterialien aus dem Textilfasergebilde ausgeschieden werden.

Die mindestens zwei Ausscheidungskriterien sind vorzugsweise von einer Reflektivität und/oder Transmissivität der Fremdmaterialien in dem betreffenden Teilbereich des elektromagnetischen Spektrums abhängig. Die mindestens zwei Ausscheidungskriterien sind vorzugsweise von einer räumlichen Ausdehnung der Fremdmaterialien abhängig.

In einer Ausführungsform ist die Referenz-Häufigkeitsverteilung von mindestens einer zuvor ermittelten Häufigkeitsverteilung der Fremdmaterialien für die Farbklassen abhängig.

In einer Ausführungsform ist die Referenz-Häufigkeitsverteilung von mindestens einer an einem anderen, gleichartigen Textilfasergebilde ermittelten Häufigkeitsverteilung der Fremdmaterialien für die Farbklassen abhängig.

Das Textilfasergebilde ist z. B. ein Faserflockenstrom oder ein Garn.

In einer Ausführungsform ist die Referenz-Häufigkeitsverteilung von mindestens einer an einem in dem textilen Herstellungsprozess nachgelagerten, aus dem Textilfasergebilde hergestellten Textilfasergebilde ermittelten Häufigkeitsverteilung der Fremdmaterialien für die Farbklassen abhängig. In diesem Fall können das Textilfasergebilde ein Faserflockenstrom und das nachgelagerte Textilfasergebilde ein Garn sein.

In einer Ausführungsform ist die Referenz-Häufigkeitsverteilung von mindestens einer an einem in dem textilen Herstellungsprozess vorgelagerten Textilfasergebilde, aus dem das Textilfasergebilde hergestellt ist, ermittelten Häufigkeitsverteilung der Fremdmaterialien für die Farbklassen abhängig. In diesem Fall können das Textilfasergebilde ein Garn und das vorgelagerte Textilfasergebilde ein Faserflockenstrom sein.

In einer Ausführungsform wird während der Wechselwirkung der elektromagnetischen Strahlung mit den Fremdmaterialien ein Bild oder je ein Bild des Textilfasergebildes in den mindestens zwei unterschiedlichen Teilbereichen des elektromagnetischen Spektrums aufgenommen. Das Bild oder die mindestens zwei Bilder werden mittels Bildverarbeitung verarbeitet. Die Fremdmaterialien werden aufgrund von Daten, die aus der entsprechenden Bildverarbeitung resultieren, detektiert.

In einer Ausführungsform besteht die Stichprobe aus mindestens 500 und vorzugsweise mindestens 5000 klassierten Fremdmaterialien.

Die erfindungsgemässe Vorrichtung dient zur Optimierung eines Garnherstellungsprozesses, in dem Fremdmaterialien in einem Textilfasergebilde überwacht werden. Sie beinhaltet mindestens eine Lichtquelle zum Anstrahlen des Textilfasergebildes mit elektromagnetischer Strahlung aus mindestens zwei unterschiedlichen Teilbereichen des elektromagnetischen Spektrums, mindestens ein Sensorsystem zur Detektion von Fremdmaterialien aufgrund ihrer Wechselwirkung mit der elektromagnetischen Strahlung und eine mit dem Sensorsystem verbundene Auswerteeinheit zur Auswertung eines Ausgangssignals des Sensorsystems. Die Auswerteeinheit ist dazu eingerichtet, jedem der mindestens zwei unterschiedlichen Teilbereiche des elektromagnetischen Spektrums eine Farbklasse von Fremdmaterialien in Abhängigkeit von der Wechselwirkung der elektromagnetischen Strahlung in dem betreffenden Teilbereich des elektromagnetischen Spektrums mit den Fremdmaterialien zuzuordnen, die detektierten Fremdmaterialien je nach ihrer Wechselwirkung mit der elektromagnetischen Strahlung in dem betreffenden Teilbereich des elektromagnetischen Spektrums in den mindestens zwei Farbklassen zu klassieren, bei Vorliegen einer Stichprobe mit einer Vielzahl von klassierten Fremdmaterialien eine Häufigkeitsverteilung der Fremdmaterialien für die Farbklassen zu ermitteln, die ermittelte Häufigkeitsverteilung mit einer Referenz-Häufigkeitsverteilung zu vergleichen und bei einer signifikanten Abweichung der ermittelten Häufigkeitsverteilung von der Referenz-Häufigkeitsverteilung mindestens eine aus einer Menge von mehreren Optimierungshandlungen automatisch auszulösen.

Die Menge von mehreren Optimierungshandlungen kann mindestens eines der folgenden Elemente beinhalten:
- ein Warnsignal wird ausgegeben;
- eine Empfehlung wird ausgegeben;
- die ermittelte Häufigkeitsverteilung wird ausgegeben.

In einer Ausführungsform beinhaltet die Vorrichtung zusätzlich eine Ausscheideeinheit zur selektiven Ausscheidung von Objekten aus dem Textilfasergebilde. Die Auswerteeinheit ist mit der Ausscheideeinheit verbunden und dazu eingerichtet, ein Ausscheidungskriterium für jede der mindestens zwei Farbklassen zu speichern, eine Ausscheidung der Fremdmaterialien aus dem Textilfasergebilde durch die Ausscheideeinheit gemäss den mindestens zwei Ausscheidungskriterien auszulösen und die Häufigkeitsverteilung derart zu ermitteln, dass sie sich auf die aus dem Textilfasergebilde ausgeschiedenen und/oder die in dem Textilfasergebilde verbliebenen Fremdmaterialien bezieht. In dieser Ausführungsform kann die Menge von mehreren Optimierungshandlungen eines der folgenden Elemente beinhalten:
- das für die betreffende Farbklasse definierte Ausscheidungskriterium wird so geändert, dass bei unverändertem Textilfasergebilde in der betreffenden Farbklasse mehr Fremdmaterialien aus dem Textilfasergebilde ausgeschieden werden;
- das für die betreffende Farbklasse definierte Ausscheidungskriterium wird so geändert, dass bei unverändertem Textilfasergebilde in der betreffenden Farbklasse weniger Fremdmaterialien aus dem Textilfasergebilde ausgeschieden werden.

Die Vorrichtung ist z. B. ein Faserreiniger oder ein Garnreiniger.

Das Sensorsystem kann mindestens eine Digitalkamera beinhalten.

Die Erfindung optimiert den Garnherstellungsprozess bezüglich Fremdmaterialien. Sie ermöglicht es, auf Änderungen des Rohmaterials und/oder auf ungeeignete Prozesseinstellungen zu reagieren. Dank der Erfindung kann die Ursache der Änderung der Farbverteilung der Fremdstoffe gezielt gesucht sowie schneller und zuverlässiger gefunden werden. Die Zuverlässigkeit ergibt sich aus der statistischen Auswertung einer genügend grossen Stichprobe, wodurch Einzelereignisse praktisch nicht ins Gewicht fallen. Ein weiterer Vorteil der Erfindung liegt darin, dass einerseits unzulässige, ausgeschiedene Fremdmaterialien, andererseits aber auch zulässige, in dem Faserflockenstrom verbleibende Fremdmaterialien berücksichtigt werden können. Auch die letzteren können z. B. wertvolle Hinweise auf Änderungen des Rohmaterials geben.

Wenn nicht ausdrücklich anders spezifiziert, so beziehen sich in dieser Schrift Ausdrücke wie "Licht", "beleuchten" und "Farbe" nicht nur auf den sichtbaren Teil des elektromagnetischen Spektrums, sondern auf das ganze elektromagnetische Spektrum, vorzugsweise auf elektromagnetische Strahlung im ultravioletten, sichtbaren und/oder infraroten Bereich. Der in dieser Schrift verwendete Begriff "Teilbereich des elektromagnetischen Spektrums" meint einen Teilbereich, der zusammenhängend oder nicht zusammenhängend sein kann.

### AUFZÄHLUNG DER ZEICHNUNGEN

Nachfolgend wird eine Ausführungsform der Erfindung anhand der Zeichnungen detailliert erläutert.
- Figur 1: zeigt schematisch eine erfindungsgemässe Vorrichtung.
- Figur 2: zeigt ein beispielhaftes Ausscheidungskriterium für Fremdmaterialien in einem Strom von Faserflocken.
- Figur 3: zeigt ein schematisches Bild eines Fremdmaterials.
- Figur 4: zeigt ein Beispiel für ein Diagramm, das zur Farbklassierung der Fremdmaterialien verwendet werden kann.
- Figur 5: zeigt zwei Beispiele für Säulendiagramme zur Darstellung von Häufigkeiten von aus dem Faserflockenstrom ausgeschiedenen Fremdmaterialien in mehreren Farbklassen.
- Figur 6: zeigt schematisch einen Teil eines Garnherstellungsprozesses in einer Spinnerei, in dem eine Ausführungsform des erfindungsgemässen Verfahrens angewendet werden kann.

### AUSFÜHRUNG DER ERFINDUNG

Nachfolgend wird nur eine mögliche Ausführungsform der Erfindung diskutiert, nämlich die Überwachung von Fremdmaterialien in einem Faserflockenstrom durch einen Faserreiniger in der Putzerei. Dies soll aber die Allgemeinheit der Erfindung keinesfalls einschränken. Alternativ kann das Textilfasergebilde ein Faserband, ein Vorgarn, ein Garn oder ein Gewebe sein. Im Fall von Garn ist die erfindungsgemässe Vorrichtung vorzugsweise ein Garnreiniger.

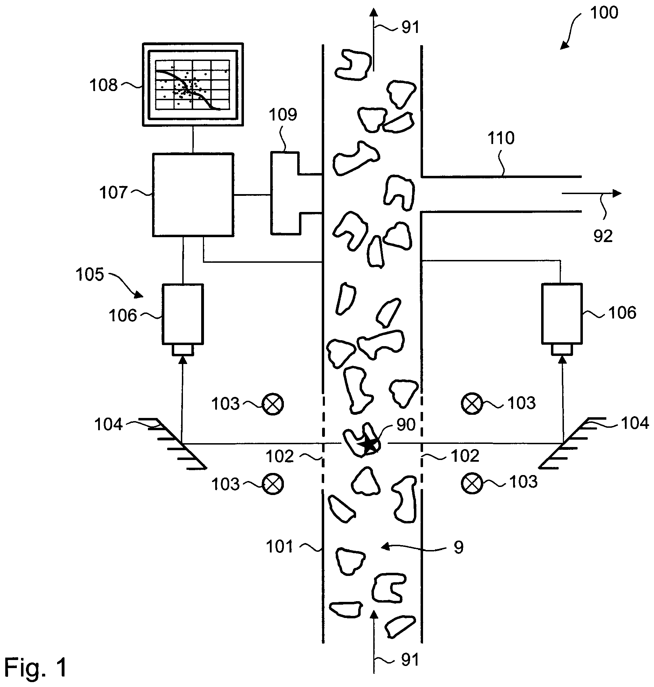

**Figur 1** zeigt schematisch eine erfindungsgemässe Vorrichtung 100. Die Vorrichtung 100 dient zur Optimierung eines Garnherstellungsprozesses. Sie überwacht Fremdmaterialien 90 in einem Textilfasergebilde 9, das im vorliegenden Beispiel ein Faserflockenstrom ist. Sie beinhaltet einen pneumatischen Fasertransportkanal 101 zum pneumatischen Transport des Faserflockenstroms 9 in einem Luftstrom. Die Transportrichtung des Faserflockenstroms 9 und des Luftstroms ist in Figur 1 durch Pfeile 91 angedeutet.

Vier Lichtquellen 103, z. B. Leuchtstoffröhren, sind in der Nähe von Fenstern 102 in einer Wand des Fasertransportkanals 101 angeordnet. Die Lichtquellen 103 beleuchten aus verschiedenen Richtungen den Faserflockenstrom 9 im Fasertransportkanal 101.

Ein Sensorsystem 105 ist am Fasertransportkanal 101 angeordnet. Es detektiert Eigenschaften von Objekten, einschliesslich Fremdmaterialien 90, im Faserflockenstrom 9. In der Ausführungsform von Figur 1 beinhaltet das Sensorsystem 105 zwei Kameras 106, z. B. CCD-Kameras, die Bilder des Faserflockenstroms 9 durch die Fenster 102 aus zwei verschiedenen Richtungen aufnehmen. Das von den Lichtquellen 103 ausgesendete Licht kann nach einer Wechselwirkung mit dem Faserflockenstrom 9 zwischen den Fenstern 102 und den Kameras 106 mittels entsprechend gekippter Spiegel 104 abgelenkt werden. Die Kameras 106 sind nur ein Beispiel für ein Sensorsystem 105; andere oder zusätzliche Sensorsysteme können in der erfindungsgemässen Vorrichtung 100 eingesetzt werden. Solche alternativen oder zusätzlichen Sensorsysteme können Eigenschaften von Objekten aufgrund von elektromagnetischen Wellen im sichtbaren oder unsichtbaren Bereich, einschliesslich Ultraviolett, Infrarot und Mikrowellen, von akustischen Wellen etc. detektieren. Andere alternative oder zusätzliche Sensorsysteme benötigen keine Lichtquellen.

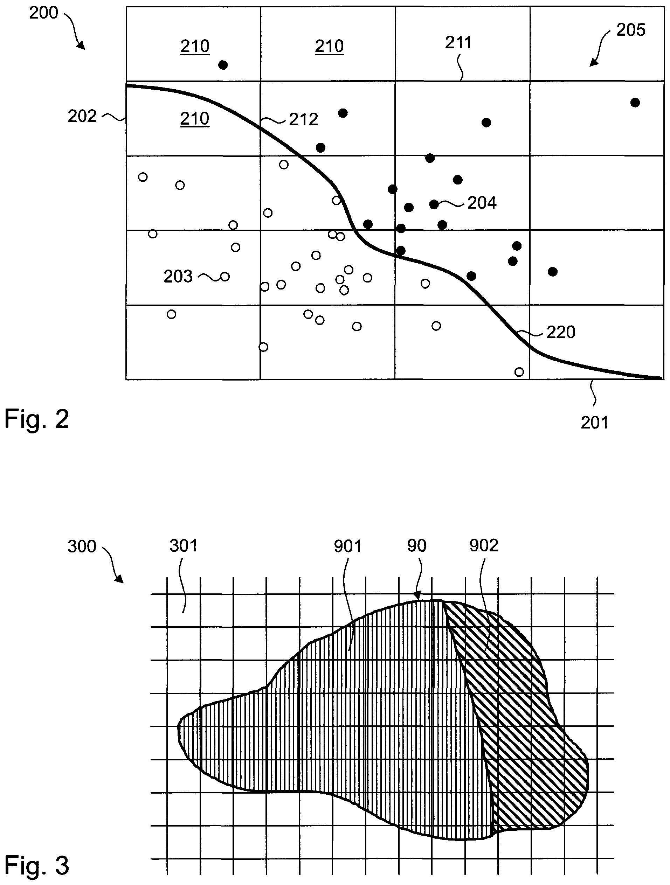

Die Kameras 106 sind mit einer Auswerteeinheit 107 zur automatischen Auswertung von Ausgangssignalen des Sensorsystems 105 verbunden. Die Auswerteeinheit 107 ist dazu eingerichtet, aus den Ausgangssignalen des Sensorsystems 105 Werte eines ersten und eines zweiten Parameters der Objekte zu ermitteln. Die Auswerteeinheit 107 ist ferner dazu eingerichtet, ein Ereignisfeld 200, wie in Figur 2 dargestellt, zur Verfügung zu stellen. Ausserdem ist die Auswerteeinheit 107 dazu eingerichtet, die für ein Objekt ermittelten ersten und zweiten Parameter als Koordinaten eines Ereignisses 203, 204 im Ereignisfeld 200 einzutragen. Die Auswerteeinheit 107 ist vorzugsweise als Computer ausgebildet.

Die Auswerteeinheit 107 ist mit einer Ausgabeeinheit 108 zur Ausgabe eines Resultates der Auswertung verbunden. Die Ausgabeeinheit 108 ist dazu eingerichtet, eine grafische Darstellung des Ereignisfeldes 200, wie in Figur 2 dargestellt, auszugeben. Die Ausgabeeinheit 108 kann z. B. ein Computerbildschirm oder ein Drucker sein. In einer Ausführungsform ist sie als Berührungsbildschirm ausgebildet und dient somit als Eingabe- und Ausgabeeinheit.

Eine Ausscheideeinheit 109 ist am Fasertransportkanal 101 stromabwärts vom Sensorsystem 105 (bezüglich der Transportrichtung 91) angeordnet. Die Ausscheideeinheit 109 dient zur selektiven Ausscheidung von Objekten aus dem Faserflockenstrom 9. Eine solche Ausscheideeinheit 109 ist an sich bekannt, z. B. aus der WO-2006/079426 A1. In einer bevorzugten Ausführungsform beinhaltet sie eine Mehrzahl von druckbeaufschlagten Luftdüsen, die einzeln durch die Auswerteeinheit 107 ansteuerbar sind. Wenn das Sensorsystem 105 ein unzulässiges Fremdmaterial 90 im Faserflockenstrom 9 detektiert, so wird die betreffende Luftdüse der Ausscheideeinheit 109 veranlasst, Druckluft senkrecht zur Transportrichtung 91 auszublasen, wenn das Fremdmaterial 90 auf der Höhe der Ausscheideeinheit 109 angekommen ist. Dadurch wird das Fremdmaterial 90 in einen Ausscheidekanal 110, der aus dem Fasertransportkanal 101 in einer Ausscheiderichtung 92, die im Wesentlichen senkrecht zur Transportrichtung 91 liegt, ausgeblasen. Die unverschmutzten Faserflocken setzen dagegen ihren Weg mit dem Faserflockenstrom 9 fort, um weiter verarbeitet zu werden.

Die Ausscheideeinheit 109 kann von der Auswerteeinheit 107 und/oder direkt vom Sensorsystem 105 angesteuert werden. Im letzteren Fall kann jeder Kamera 106 ein Mikroprozessor zugeordnet sein, und die Kameras 106 können direkt mit der Ausscheideeinheit 109 verbunden sein. Solche direkten Verbindungen sind in Figur 1 der Einfachheit halber nicht eingezeichnet. In einer weiteren Alternative wird die Ausscheideeinheit 109 durch einen Mikroprozessor angesteuert, welcher der Ausscheideeinheit 109 selbst zugeordnet ist.

Wie oben erwähnt, kann eine grafische Darstellung des Ereignisfeldes 200, welches durch die Auswerteeinheit 107 zur Verfügung gestellt wird, auf der Ausgabeeinheit 108 ausgegeben werden. Ein Beispiel einer grafischen Darstellung des Ereignisfeldes 200 ist in **Figur 2** gezeigt. Das Ereignisfeld 200 beinhaltet einen Quadranten oder einen Teil eines Quadranten eines zweidimensionalen Kartesischen Koordinatensystems. Entlang einer ersten Achse 201, z. B. der Abszisse, ist der erste Parameter und entlang einer zweiten Achse 202, z. B. der Ordinate, ist der zweite Parameter aufgetragen. Der erste Parameter kann sich auf eine geometrische Eigenschaft der Objekte beziehen und ist vorzugsweise eine Länge oder ein Flächeninhalt der Objekte. Der zweite Parameter kann sich auf eine optische Eigenschaft der Objekte beziehen und ist vorzugsweise eine Intensität von Licht, das durch die Objekte reflektiert und/oder transmittiert wird. Die Werte des ersten Parameters und des zweiten Parameters, die für ein Objekt bestimmt wurden, werden in das Ereignisfeld 200 als Koordinaten des Objektes eingetragen. So wird ein Objekt durch ein grafisches Symbol 203, 204, z. B. einen Punkt, dargestellt, das an dem Ort liegt, der den Koordinaten des Objektes entspricht. Eine solche Darstellung eines Objektes im Ereignisfeld 200 wird nachfolgend ein "Ereignis" 203, 204 genannt. Eine Vielzahl von Ereignissen 203, 204 erzeugt eine Punktewolke 205, welche die Koordinaten der entsprechenden Objekte zeigt.

In einer Ausführungsform der Erfindung werden mindestens zwei Objektklassen von Objekten in Form von nichtüberlappenden Flächen 210 in dem Ereignisfeld 200 vorbestimmt. Im Beispiel von Figur 2 sind die Flächen 210 benachbarte Rechtecke, die voneinander durch Geraden 211, 212, die zur ersten Achse 201 bzw. zur zweiten Achse 202 parallel sind, abgegrenzt sind. In der beispielhaften Ausführungsform von Figur 2 gibt es 4×5 = 20 Objektklassen 210; andere Objektklassierungen mit anderen Formen und/oder einer anderen Anzahl Objektklassen 210 sind möglich. Ein Ereignis 203, 204 wird in einer der mindestens zwei Objektklassen 210 klassiert, wenn die Koordinaten des Ereignisses 203, 204 in der betreffenden Fläche 210 liegen. Ereignisse 203, 204, die in mindestens einer der mindestens zwei Objektklassen 210 liegen, werden gezählt, und die einzelnen gezählten Anzahlen von Ereignissen 203, 204 werden für jede der mindestens einen Objektklasse 210 ausgegeben. Die gezählten Anzahlen von Ereignissen 203, 204 können anstelle oder zusätzlich zu der grafischen Darstellung von Figur 2 ausgegeben werden. Die Objektklassierung hilft, die Fremdmaterialien 90, die im Faserflockenstrom 9 enthalten sind, numerisch zu charakterisieren. Sie ist von der weiter unten beschriebenen Farbklassierung zu unterscheiden.

Eine Ausscheidungskurve 220, die ein Ausscheidungskriterium für Fremdmaterialien darstellt, kann in dem Ereignisfeld 200 eingezeichnet und zusammen mit dem Ereignisfeld 200 ausgegeben werden. Das Ausscheidungskriterium wird als Kriterium für die Zulässigkeit oder Unzulässigkeit der Objekte vorbestimmt. Objekte, die durch Ereignisse 203 mit Koordinaten auf der einen Seite der Ausscheidungskurve 220 dargestellt sind, werden in dem Faserflockenstrom 9 belassen, wohingegen Objekte, die durch Ereignisse 204 mit Koordinaten auf der anderen Seite der Ausscheidungskurve 220 dargestellt sind, werden aus dem Faserflockenstrom 9 ausgeschieden. Ereignisse 203, 204, die den zulässigen bzw. unzulässigen Objekten entsprechen, können durch unterschiedliche grafische Symbole dargestellt werden, bspw. durch unterschiedliche Formen, unterschiedliche Farben und/oder unterschiedliche Füllungen. In der beispielhaften Ausführungsform von Figur 2 sind zulässige Ereignisse 203 durch leere Kreise dargestellt, während unzulässige Ereignisse 204 durch ausgefüllte Kreise dargestellt sind.

Das Ausscheidungskriterium kann durch eine Eingabe einer Bedienperson vorbestimmt werden, kann einer Datenbank, die verschiedene Typen von Ausscheidungskriterien enthält, entnommen werden oder kann automatisch berechnet werden.

In der beispielhaften Ausführungsform von Figur 2 ist die Ausscheidungskurve 220 unabhängig von den Objektklassen 210 und kann somit von einer Bedienperson im Wesentlichen frei definiert werden. Alternativ kann die Ausscheidungskurve 220 den Objektklassengrenzen 211, 212 folgen.

Für die folgenden Erläuterungen der Erfindung wird der Einfachheit halber angenommen, dass jede der Kameras 106 (Figur 1) dazu eingerichtet ist, rotes und grünes Licht zu empfangen und voneinander zu unterscheiden. Dies kann z. B. erreicht werden, indem die Kamera 106 mit einem digitalen Empfängersensor ausgestattet ist, der eine Vielzahl von Bildelementen, sog. Pixeln, enthält, von denen ein Teil für rotes und ein anderer Teil für grünes Licht empfindlich ist. Zu diesem Zweck können die Pixel z. B. mit rotdurchlässigen bzw. gründurchlässigen Filtern versehen sein. Solche Digitalkameras sind bekannt, wobei üblicherweise ein weiterer Teil der Pixel für blaues Licht empfindlich ist (RGB-Kameras). Ferner wird angenommen, dass die Lichtquellen 103 rotes und grünes Licht aussenden. Die Lichtquellen 103 können breitbandige Weisslichtquellen sein. Alternativ kann es sich bei den Lichtquellen 103 um schmalbandige Lichtquellen handeln, von denen ein Teil nur rotes und ein anderer Teil nur grünes Licht aussendet. In einer anderen Ausführungsform können die Lichtquellen 103 periodisch abwechslungsweise rotes bzw. grünes Licht aussenden.

**Figur 3** zeigt schematisch ein Bild 300 eines Fremdmaterials 90, wie es im Faserflockenstrom 9 auftreten kann. Das Bild 300 wird von einer der oder von beiden Kameras 106 (Figur 1) aufgenommen. Ohne Einschränkung der Allgemeinheit sei angenommen, das Bild 300 sei im Auflicht aufgenommen, d. h. mit vom Fremdmaterial 90 reflektiertem Licht. Das Raster deutet die Pixel 301 an, aus denen das Bild 300 zusammengesetzt ist. Das Fremdmaterial 90 kann verschiedenfarbig sein. Im Beispiel von Figur 3 besteht es aus einem ersten Bereich 901, der rotes Licht reflektiert, und einem zweiten Bereich 902, der grünes Licht reflektiert.

Das Bild 300 wird mittels Bildverarbeitung verarbeitet. Die Bildverarbeitung kann in der oder den Kameras 106 selbst, in einer anderen Komponente des Sensorsystems 105 und/oder in der Auswerteeinheit 107 erfolgen. Der nachfolgend beschriebenen Auswertung werden Daten, die aus der Bildverarbeitung resultieren, zugrunde gelegt. Die Auswertung erfolgt vorzugsweise in der Auswerteeinheit 107.

Die Fremdmaterialien 90 werden automatisch in Farbklassen klassiert. Jeder detektierten Farbe, im vorliegenden Beispiel Rot und Grün, wird eine Farbklasse zugeordnet. Ein mehrfarbiges Fremdmaterial 90 kann in diejenige Farbklasse klassiert werden, die der Farbe des Grossteils seiner Fläche entspricht, oder der Mehrheit der Pixel 301 seines Bildes 300. Im Beispiel von Figur 3 wird das Fremdmaterial 90 also in die Farbklasse "Rot" klassiert. Alternative Klassierverfahren sind möglich, z. B. nach der Farbe der grössten zusammenhängenden Fläche.

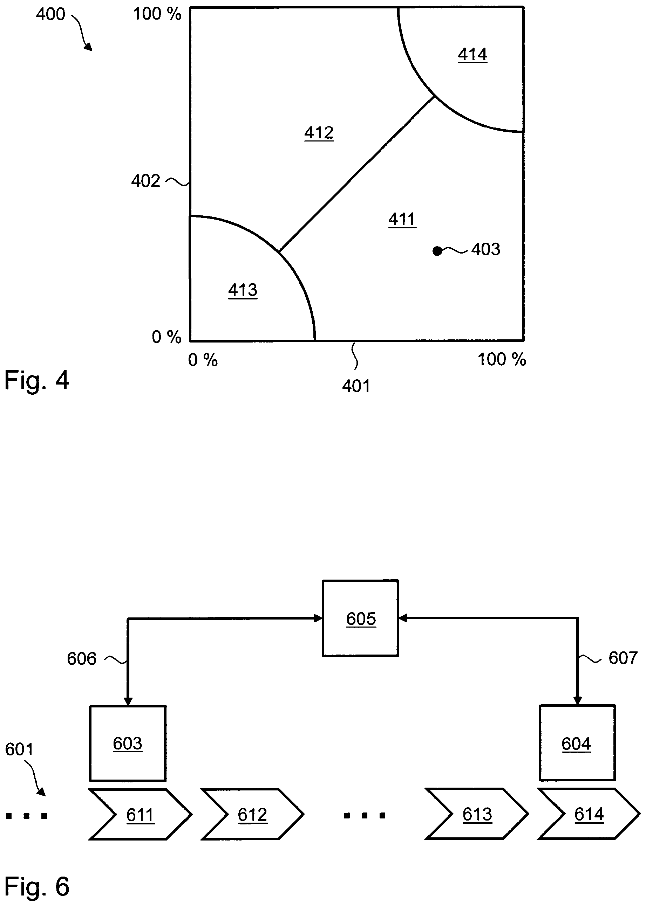

Ein Beispiel für ein Diagramm 400, das zur Farbklassierung der Fremdmaterialien 90 verwendet werden kann, ist in **Figur 4** dargestellt. Das quadratische Diagramm 400 wird durch zwei zueinander senkrechte Achsen 401, 402 aufgespannt. Entlang einer ersten Achse 401 wird ein Anteil der Farbe Rot, entlang einer zweiten Achse 402 ein Anteil der Farbe Grün an der Fläche eines jeweiligen Fremdmaterials 90 aufgetragen. Das Diagramm 400 ist in vier Farbklassen 411-414 aufgeteilt: eine erste Farbklasse 411 für rote Fremdmaterialien 90, eine zweite Farbklasse 412 für grüne Fremdmaterialien 90, eine dritte Farbklasse 413 für blaue/schwarze Fremdmaterialien 90 und eine vierte Farbklasse 414 für gelbe/weisse Fremdmaterialien. Im Bild des Fremdmaterials 90 (siehe Figur 3) können für jede detektierte Farbe, im vorliegenden Beispiel Rot und Grün, die Pixel mit der betreffenden Farbe gezählt und für jede detektierte Farbe ein Anteil der Pixel an der Gesamtzahl der Pixel bestimmt werden. Die beiden Anteile definieren Koordinaten eines Punktes 403 in dem Diagramm 400, der das Fremdmaterial 90 darstellt. Das Fremdmaterial 90 wird in diejenige Farbklasse klassiert, in welcher sein Punkt 403 liegt, im Beispiel von Figur 4 also in die erste Farbklasse 411 "Rot".

Für jede Farbklasse 411-414 wird ein Ausscheidungskriterium definiert, wie es in Figur 2 durch die Ausscheidungskurve 220 dargestellt ist. Fremdmaterialien 90 werden je nach dem für ihre Farbklasse 411-414 definierten Ausscheidungskriterium aus dem Faserflockenstrom 9 ausgeschieden oder in dem Faserflockenstrom 9 belassen.

Wenn eine statistisch relevante Stichprobe von klassierten Fremdmaterialien 90 vorliegt, wird für die Farbklassen 411-414 jeweils automatisch eine Häufigkeitsverteilung der Fremdmaterialien 90 bestimmt. Die Häufigkeitsverteilung kann sich auf alle detektierten, auf die aus dem Faserflockenstrom 9 ausgeschiedenen und/oder auf die im Faserflockenstrom 9 verbliebenen Fremdmaterialien 90 beziehen. Im hier diskutierten Beispiel werden ohne Einschränkung der Allgemeinheit die Häufigkeiten der aus dem Faserflockenstrom 9 ausgeschiedenen Fremdmaterialien 90 betrachtet.

Der für die statistische Relevanz benötigte Umfang der Stichprobe von klassierten Fremdmaterialien 90 hängt von den jeweiligen Umständen ab. In vielen Fällen wird es genügen, ihn empirisch festzulegen. In einer ersten Ausführungsform kann ein fester Wert für den Stichprobenumfang vorgegeben werden, z. B. mindestens 500 und vorzugsweise mindestens 5000 Fremdmaterialien 90. In einer zweiten Ausführungsform kann die Stichprobe aus allen innerhalb eines genügend grossen Zeitintervalls, z. B. einer Stunde, klassierten Fremdmaterialien 90 bestehen. Das Zeitintervall kann zeitlich nachgeführt werden. In einer dritten Ausführungsform kann die Stichprobe aus allen innerhalb einer genügend grossen Masse, z. B. 500 kg, des Faserflockenstroms 9 klassierten Fremdmaterialien 90 bestehen. Weitere, komplexere Verfahren zur Bestimmung des benötigten Stichprobenumfangs kann der Fachmann der statistischen Literatur entnehmen.

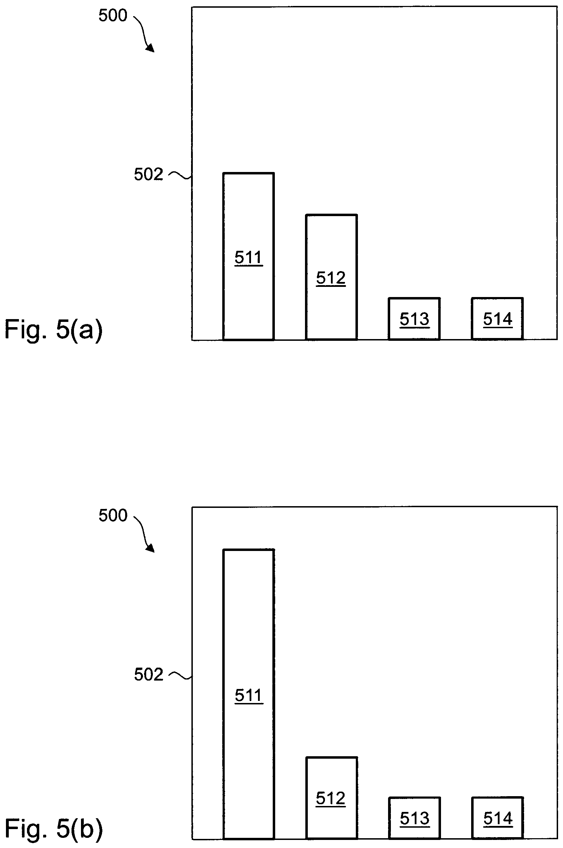

In **Figur 5** sind beispielhafte Häufigkeitsverteilungen der aus dem Faserflockenstrom 9 ausgeschiedenen Fremdmaterialien 90 in mehreren Farbklassen 411-414 in Säulendiagrammen 500 dargestellt. Entlang einer vertikalen Achse 502 des jeweiligen Säulendiagramms 500 ist eine relative oder absolute Häufigkeit der Fremdmaterialien 90 aufgetragen. Die Fläche oder Höhe einer Säule gibt jeweils einen Anteil oder eine Häufigkeit 511-514 der aus dem Faserflockenstrom 9 ausgeschiedenen Fremdmaterialien 90 an. Die Häufigkeiten 511-514 entsprechen den Farbklassen 411-414: eine erste Häufigkeit 511 ausgeschiedener roter Fremdmaterialien 90, eine zweite Häufigkeit 512 ausgeschiedener grüner Fremdmaterialien 90, eine dritte Häufigkeit 513 ausgeschiedener blauer/schwarzer Fremdmaterialien 90 und eine vierte Häufigkeit 514 ausgeschiedener gelber/weisser Fremdmaterialien 90.

Die beiden Säulendiagramme 500 der Figuren 5(a) und 5(b) können z. B. die Häufigkeiten 511-514 angeben, die an derselben Vorrichtung 100 zu verschiedenen Zeitpunkten bestimmt wurden. Die Häufigkeitsverteilung 511-514 zu einem ersten Zeitpunkt gemäss Figur 5(a) kann als Referenz-Häufigkeitsverteilung dienen. Die Häufigkeitsverteilung 511-514 zu einem zweiten, späteren Zeitpunkt gemäss Figur 5(b) wird mit der Referenz-Häufigkeitsverteilung verglichen. Offensichtlich weicht im vorliegenden Beispiel die Häufigkeitsverteilung 511-514 gemäss Figur 5(b) signifikant von der Referenz-Häufigkeitsverteilung gemäss Figur 5(a) ab, insbesondere indem sich die erste Häufigkeit 511 ausgeschiedener roter Fremdmaterialien 90 zulasten der zweiten Häufigkeit 512 ausgeschiedener grüner Fremdmaterialien 90 vergrössert hat. Bei einer solchen signifikanten Abweichung wird erfindungsgemäss eine Optimierungshandlung automatisch vorgenommen.

Die Optimierungshandlung kann z. B. in einer Ausgabe eines optischen und/oder akustischen Warnsignals bestehen. Mit dem Warnsignal wird eine Bedienperson auf die Abweichung aufmerksam gemacht. Zusätzlich oder alternativ zum Warnsignal kann die Häufigkeitsverteilung an die Bedienperson ausgegeben werden, z. B. in grafischer Form wie in Figur 5 auf der Ausgabeeinheit 108 (Figur 1). Zusätzlich oder alternativ zum Warnsignal und/oder zur Ausgabe der Häufigkeitsverteilung kann automatisch eine Handlungsempfehlung an die Bedienperson ausgegeben werden, z. B. "Ausscheidungskriterium für die Farbklasse ,Rot' empfindlicher einstellen!" Daraufhin kann die Bedienperson das Ausscheidungskriterium für die erste Farbklasse "Rot" 411 empfindlicher stellen, so dass bei unverändertem Faserflockenstrom 9 in der ersten Farbklasse "Rot" 411 mehr Fremdmaterialien 90 aus dem Faserflockenstrom 9 ausgeschieden werden. Alternativ kann die Änderung des Ausscheidungskriteriums von der Auswerteeinheit 107 automatisch vorgenommen werden.

In der obigen Ausführungsform ist die Referenz-Häufigkeitsverteilung gemäss Figur 5(a) die zuvor an derselben Vorrichtung 100 bestimmte Häufigkeitsverteilung 511-514 der ausgeschiedenen Fremdmaterialien 90. In einer anderen Ausführungsform kann die Referenz-Häufigkeitsverteilung von einer an einer anderen Vorrichtung bestimmten Häufigkeitsverteilung der aus einem anderen Faserflockenstrom ausgeschiedenen Fremdmaterialien abhängig sein. So kann z. B. die Referenz-Häufigkeitsverteilung von genau einer gleichzeitig laufenden erfindungsgemässen Vorrichtung stammen oder ein Mittelwert von Häufigkeitsverteilungen mehrerer gleichzeitig laufender erfindungsgemässer Vorrichtungen sein.

Damit die Optimierungshandlung ausgelöst wird, muss die Abweichung der Häufigkeitsverteilung 511-514 von der entsprechenden Referenz-Häufigkeitsverteilung signifikant, d. h. genügend gross, sein. Wann dies der Fall ist, kann je nach Umständen festgelegt werden. In einer ersten Alternative können einzelne Häufigkeiten 511 miteinander verglichen werden und z. B. ein Schwellwert für die Abweichung vorgegeben werden, bspw. als Bruchteil der Referenz-Häufigkeit oder als fester Wert auf der vertikalen Achse 502. In einer zweiten Alternative können Verhältnisse von Häufigkeiten betrachtet werden, z. B. das Verhältnis der ersten Häufigkeit 511 zur zweiten Häufigkeit 512. Weitere, komplexere Verfahren zur Feststellung von Signifikanz kann der Fachmann der statistischen Literatur entnehmen.

Die Säulendiagramme 500 von Figur 5 sind nur eine von vielen Möglichkeiten, die Häufigkeitsverteilung 511-514 der Fremdmaterialien 90 darzustellen. Andere infrage kommende Diagrammtypen wie z. B. ein Kreisdiagramm sind bekannt. Falls ein zeitlicher Verlauf der Häufigkeitsverteilung 511-514 betrachtet wird, so kann ein Liniendiagramm vorteilhaft sein. Die Darstellung kann auf der Ausgabeeinheit 108 (Figur 1) an eine Bedienperson ausgegeben werden. In anderen Ausführungsformen braucht die Häufigkeitsverteilung 511-514 gar nicht dargestellt und ausgegeben zu werden. Eine Ausgabe eines Warnsignals zusammen mit einer entsprechenden Begründung oder einer entsprechenden Handlungsempfehlung kann genügen. Die Darstellung kann auch dann entfallen, wenn die Änderung des Ausscheidungskriteriums automatisch vorgenommen wird.

Das obige Beispiel mit nur zwei Farben Rot und Grün dient einer einfachen Erläuterung der Erfindung. Selbstverständlich umfasst die Erfindung auch Ausführungsformen mit mehr als zwei Farben, bspw. mit den drei Farben Rot, Grün und Blau. Im letztgenannten Fall kann das zweidimensionale Diagramm 400 von Figur 4 auf den dreidimensionalen RGB-Farbraum erweitert werden. Alternativ können andere Farbsysteme wie z. B. ein Farbkreis verwendet werden. Die "Farben" brauchen nicht schmalbandige Bereiche des sichtbaren Spektrums zu sein, sondern können unterschiedliche Teilbereiche des elektromagnetischen Spektrums sein, die jeweils nicht zusammenhängend zu sein brauchen.

**Figur 6** zeigt schematisch einen Teil eines Garnherstellungsprozesses 601, der in einer Spinnerei abläuft. In dem Garnherstellungsprozess 601 wird z. B. aus Rohbaumwolle Garn gesponnen. Der Garnherstellungsprozess 601 kann z. B. die folgenden Prozessschritte beinhalten: Öffnen, Grobreinigen, Mischen, Feinreinigen 611, Kardieren 612, Doublieren, Kämmen, Verstrecken, Verspinnen 613, Umspulen 614. Nicht alle genannten Prozessschritte 611-614 brauchen durchlaufen zu werden, und es können weitere Prozessschritte hinzukommen. Der Einfachheit halber sind in Figur 6 nur einige wenige Prozessschritte 611-614 schematisch eingezeichnet, während andere durch Punkte angedeutet sind.

An einer ersten Stelle in einem frühen Stadium des Garnherstellungsprozesses 601, z. B. in oder unmittelbar nach der Feinreinigung 611, liegt ein Faserflockenstrom 9 (Figur 1) vor, die pneumatisch in einem Luftstrom gefördert werden. An dieser ersten Stelle befindet sich eine erfindungsgemässe Vorrichtung, die in Figur 6 mit dem Bezugszeichen 603 versehen ist.

An einer zweiten Stelle des Garnherstellungsprozesses 601, die stromabwärts bezüglich der ersten Stelle liegt, wird Garn, das aus den Faserflocken gesponnen wurde, entlang seiner Längsrichtung gefördert, z. B. während des Umspulens 614. An dieser zweiten Stelle befindet sich eine Garnüberwachungseinrichtung 604. Die Garnüberwachungseinrichtung 604 ist dazu eingerichtet, das Garn auf Fremdmaterialien zu überwachen. Sie kann z. B. als ein Garnreinigersystem ausgeführt sein. Garnreiniger zur optischen Überwachung eines laufenden Garns auf Fremdmaterialien sind an sich bekannt, z. B. aus der WO-2011/026249 A1. Dementsprechend beinhaltet die Garnüberwachungseinrichtung 604 ein Sensorsystem, das Messwerte einer optischen Messung an einem Garnabschnitt entlang der Längsrichtung des Garns erfasst. Das Sensorsystem ist dazu eingerichtet, Fremdmaterialien der mindestens zwei Farben, denen die Farbklassen 411-414 zugeordnet sind, zu erkennen und voneinander zu unterscheiden. Die Garnüberwachungseinrichtung 604 beinhaltet ferner eine Auswerteeinheit zur Ermittlung von Werten einer Reflektivität des ausgemessenen Garnabschnitts aus den Messwerten. Die Auswerteeinheit klassiert die Fremdmaterialien im Garn in die mindestens zwei Farbklassen 411-414 und bestimmt Häufigkeiten, bezüglich aller Farbklassen 411-414, der Fremdmaterialien in den mindestens zwei Farbklassen 411-414.

In der Ausführungsform gemäss Figur 6 ist die Garnüberwachungseinrichtung 604 mit einer zentralen Steuereinrichtung 605 verbunden, was durch einen Pfeil 607 dargestellt ist. Die zentrale Steuereinrichtung 605 ist ihrerseits mit der erfindungsgemässen Vorrichtung 603 verbunden, was durch einen Pfeil 606 dargestellt ist. Die Datenverbindungen 606, 607 ermöglichen einen Austausch von Daten zwischen den jeweils beteiligten Einrichtungen 603, 604, 605. Es ist vorteilhaft, wenn beide Datenverbindungen 606, 607 für einen bidirektionalen Datenaustausch eingerichtet sind. Zu diesem Zweck sind die erfindungsgemässe Vorrichtung 603 und die Garnüberwachungseinrichtung 604 jeweils mit Sendemitteln zum Senden von Daten und mit Empfangsmitteln zum Empfangen von Daten ausgestattet. Die Datenverbindungen 606, 607 können kabelgebunden oder kabellos ausgeführt sein.

Die zentrale Steuereinrichtung 605 kann als ein eigenständiges Gerät ausgeführt sein, z. B. als ein Computer, der sich in der Spinnerei oder ausserhalb der Spinnerei befindet. Sie beinhaltet entsprechende Empfangs- und Sendemittel zum Empfangen bzw. Senden von Daten. Alternativ kann die zentrale Steuereinrichtung 605 in einem anderen Gerät integriert sein, z. B. in einem Garnprüfgerät im Textillabor der Spinnerei, in der erfindungsgemässen Vorrichtung 603, in der Garnüberwachungseinrichtung 604 etc. In den letzteren beiden Fällen kann eine direkte Datenverbindung zwischen der Garnüberwachungseinrichtung 604 der erfindungsgemässen Vorrichtung 603 bestehen, über welche die beiden Einrichtungen 604, 603 Daten übermitteln oder austauschen.

Entlang der Verbindung 606 und/oder 607 können sich weitere (nicht eingezeichnete) Einrichtungen befinden, welche die übermittelten Daten empfangen, bei Bedarf verarbeiten und weitersenden. In einer Ausführungsform sind mehrere erfindungsgemässe Vorrichtungen 603 mit einem Faserflockenexpertensystem verbunden. Das Faserflockenexpertensystem ist dazu eingerichtet, Daten von den erfindungsgemässen Vorrichtungen 603 zu empfangen, zu verarbeiten und in geeigneter Form auszugeben, sowie die erfindungsgemässen Vorrichtungen 603 zu steuern. Es ist seinerseits mit der zentralen Steuereinrichtung 605 verbunden. In einer Ausführungsform sind mehrere Garnüberwachungseinrichtungen 604 mit einem Garnexpertensystem verbunden. Das Garnexpertensystem ist dazu eingerichtet, Daten von den Garnüberwachungseinrichtungen 604 zu empfangen, zu verarbeiten und in geeigneter Form auszugeben, sowie die Garnüberwachungseinrichtungen 604 zu steuern. Es ist seinerseits mit der zentralen Steuereinrichtung 605 verbunden.

In einer Ausführungsform des erfindungsgemässen Verfahrens ist die Referenz-Häufigkeitsverteilung von der an einem aus dem Faserflockenstrom 9 hergestellten Garn bestimmten Häufigkeitsverteilung der in dem Faserflockenstrom 9 verbliebenen Fremdmaterialien 90 abhängig. Aufgrund der aktuellen Referenz-Häufigkeitsverteilung wird das für die betreffende Farbklasse 411-414 definierte Ausscheidungskriterium geändert, und vorzugsweise automatisch geändert. Somit wird die Häufigkeitsverteilung der in dem Faserflockenstrom 9 verbliebenen Fremdmaterialien 90 geregelt. Es liegt ein Regelkreis vor, in dem die erfindungsgemässe Vorrichtung 603 die Regelstrecke, die zentrale Steuereinrichtung 605 der Regler und die Datenverbindung 607 die Rückkopplung sind.

### BEZUGSZEICHENLISTE

- 100: erfindungsgemässe Vorrichtung
- 101: Fasertransportkanal
- 102: Fenster in Wand des Fasertransportkanals
- 103: Lichtquellen
- 104: Spiegel
- 105: Sensorsystem
- 106: Kameras
- 107: Auswerteeinheit
- 108: Ausgabeeinheit
- 109: Ausscheideeinheit
- 110: Ausscheidekanal

- 200: Ereignisfeld
- 201, 202: erste bzw. zweite Achse des Ereignisfeldes
- 203: zulässiges Ereignis
- 204: unzulässiges Ereignis
- 205: Punktewolke
- 210: Flächen im Ereignisfeld, die Objektklassen darstellen
- 211: horizontale Klassengrenzen
- 212: vertikale Klassengrenzen
- 220: Ausscheidungskurve

- 300: Bild eines Fremdmaterials
- 301: Pixel

- 400: Diagramm zur Farbklassierung
- 401, 402: erste bzw. zweite Achse des Diagramms
- 403: Punkt im Diagramm, der das Fremdmaterial darstellt
- 411-414: Farbklassen

- 500: Säulendiagramm
- 502: vertikale Achse des Säulendiagramms
- 511-514: Häufigkeiten von Fremdmaterialien

- 601: Garnherstellungsprozess
- 603: erfindungsgemässe Vorrichtung
- 604: Garnüberwachungseinrichtung
- 605: zentrale Steuereinrichtung
- 606, 607: Datenverbindungen
- 611: Feinreinigen
- 612: Kardieren
- 613: Verspinnen
- 614: Umspulen

- 9: Faserflockenstrom
- 90: Fremdmaterial
- 91: Transportrichtung des Faserflockenstroms
- 92: Ausscheiderichtung
- 901: roter Bereich des Fremdmaterials
- 902: grüner Bereich des Fremdmaterials

## Patentansprüche

1. Verfahren zur Optimierung eines Garnherstellungsprozesses, in dem Fremdmaterialien (90) in einem Textilfasergebilde (9), bspw. einem Faserflockenstrom oder einem Garn, überwacht werden, wobei das Textilfasergebilde (9) mit elektromagnetischer Strahlung aus mindestens zwei unterschiedlichen Teilbereichen des elektromagnetischen Spektrums angestrahlt wird,
die elektromagnetische Strahlung mit den Fremdmaterialien (90) wechselwirkt und Fremdmaterialien (90) aufgrund ihrer Wechselwirkung mit der elektromagnetischen Strahlung detektiert werden,
**dadurch gekennzeichnet, dass**
jedem der mindestens zwei unterschiedlichen Teilbereiche des elektromagnetischen Spektrums eine Farbklasse (411-414) von Fremdmaterialien (90) in Abhängigkeit von der Wechselwirkung der elektromagnetischen Strahlung in dem betreffenden Teilbereich des elektromagnetischen Spektrums mit den Fremdmaterialien (90) zugeordnet wird,
die detektierten Fremdmaterialien (90) je nach ihrer Wechselwirkung mit der elektromagnetischen Strahlung in dem betreffenden Teilbereich des elektromagnetischen Spektrums automatisch in den mindestens zwei Farbklassen (411-414) klassiert werden,
bei Vorliegen einer Stichprobe mit einer Vielzahl von klassierten Fremdmaterialien (90) automatisch eine Häufigkeitsverteilung (511-514) der Fremdmaterialien (90) für die Farbklassen (411-414) ermittelt wird,
die ermittelte Häufigkeitsverteilung (511-514) automatisch mit einer Referenz-Häufigkeitsverteilung verglichen wird und
bei einer Abweichung der ermittelten Häufigkeitsverteilung (511-514) von der Referenz-Häufigkeitsverteilung mindestens eine aus einer Menge von mehreren Optimierungshandlungen automatisch vorgenommen wird.

2. Verfahren nach Anspruch 1, wobei die Menge von mehreren Optimierungshandlungen mindestens eines der folgenden Elemente beinhaltet:
• ein Warnsignal wird ausgegeben;
• eine Empfehlung wird ausgegeben;
• die ermittelte Häufigkeitsverteilung (511-514) wird ausgegeben.

3. Verfahren nach Anspruch 1 oder 2, wobei
für jede der mindestens zwei Farbklassen (411-414) ein Ausscheidungskriterium definiert wird,
Fremdmaterialien (90) gemäss den mindestens zwei Ausscheidungskriterien aus dem Textilfasergebilde (9) ausgeschieden werden und
die Häufigkeitsverteilung sich auf die aus dem Textilfasergebilde (9) ausgeschiedenen und/oder die in dem Textilfasergebilde (9) verbliebenen Fremdmaterialien (90) bezieht.

4. Verfahren nach Anspruch 3, wobei die Menge von mehreren Optimierungshandlungen eines der folgenden Elemente beinhaltet:
• das für die betreffende Farbklasse (411-414) definierte Ausscheidungskriterium wird so geändert, dass bei unverändertem Textilfasergebilde (9) in der betreffenden Farbklasse (411-414) mehr Fremdmaterialien (90) aus dem Textilfasergebilde (9) ausgeschieden werden;
• das für die betreffende Farbklasse (411-414) definierte Ausscheidungskriterium wird so geändert, dass bei unverändertem Textilfasergebilde (9) in der betreffenden Farbklasse (411-414) weniger Fremdmaterialien (90) aus dem Textilfasergebilde (9) ausgeschieden werden.

5. Verfahren nach Anspruch 3 oder 4, wobei die mindestens zwei Ausscheidungskriterien von einer Reflektivität und/oder Transmissivität der Fremdmaterialien (90) in dem betreffenden Teilbereich des elektromagnetischen Spektrums abhängig sind.

6. Verfahren nach einem der Ansprüche 3-5, wobei die mindestens zwei Ausscheidungskriterien von einer räumlichen Ausdehnung der Fremdmaterialien (90) abhängig sind.

7. Verfahren nach einem der vorangehenden Ansprüche, wobei die Referenz-Häufigkeitsverteilung von mindestens einer zuvor ermittelten Häufigkeitsverteilung (511-514) der Fremdmaterialien (90) für die Farbklassen (411-414) abhängig ist.

8. Verfahren nach einem der Ansprüche 1-6, wobei die Referenz-Häufigkeitsverteilung von mindestens einer an einem anderen, gleichartigen Textilfasergebilde ermittelten Häufigkeitsverteilung der Fremdmaterialien für die Farbklassen (411-414) abhängig ist.

9. Verfahren nach einem der Ansprüche 1-6, wobei die Referenz-Häufigkeitsverteilung von mindestens einer an einem in dem textilen Herstellungsprozess nachgelagerten, aus dem Textilfasergebilde (9) hergestellten Textilfasergebilde ermittelten Häufigkeitsverteilung der Fremdmaterialien für die Farbklassen (411-414) abhängig ist und vorzugsweise das Textilfasergebilde (9) ein Faserflockenstrom und das nachgelagerte Textilfasergebilde ein Garn ist.

10. Verfahren nach einem der Ansprüche 1-6, wobei die Referenz-Häufigkeitsverteilung von mindestens einer an einem in dem textilen Herstellungsprozess vorgelagerten Textilfasergebilde, aus dem das Textilfasergebilde (9) hergestellt ist, ermittelten Häufigkeitsverteilung der Fremdmaterialien für die Farbklassen (411-414) abhängig ist und vorzugsweise das Textilfasergebilde (9) ein Garn und das vorgelagerte Textilfasergebilde ein Faserflockenstrom ist.

11. Verfahren nach einem der vorangehenden Ansprüche, wobei
während der Wechselwirkung der elektromagnetischen Strahlung mit den Fremdmaterialien (90) ein Bild (300) oder je ein Bild des Textilfasergebildes (9) in den mindestens zwei unterschiedlichen Teilbereichen des elektromagnetischen Spektrums aufgenommen wird,
das Bild (300) oder die mindestens zwei Bilder mittels Bildverarbeitung verarbeitet werden und
die Fremdmaterialien (90) aufgrund von Daten, die aus der entsprechenden Bildverarbeitung resultieren, detektiert werden.

12. Vorrichtung (100), bspw. ein Faserreiniger oder ein Garnreiniger, zur Optimierung eines Garnherstellungsprozesses, in dem Fremdmaterialien (90) in einem Textilfasergebilde (9) überwacht werden, beinhaltend
mindestens eine Lichtquelle (103) zum Anstrahlen des Textilfasergebildes (9) mit elektromagnetischer Strahlung aus mindestens zwei unterschiedlichen Teilbereichen des elektromagnetischen Spektrums,
mindestens ein Sensorsystem (105) zur Detektion von Fremdmaterialien (90) aufgrund ihrer Wechselwirkung mit der elektromagnetischen Strahlung und
eine mit dem Sensorsystem (105) verbundene Auswerteeinheit (107) zur Auswertung eines Ausgangssignals des Sensorsystems (105),
**dadurch gekennzeichnet, dass**
die Auswerteeinheit (107) dazu eingerichtet ist,
jedem der mindestens zwei unterschiedlichen Teilbereiche des elektromagnetischen Spektrums eine Farbklasse (411-414) von Fremdmaterialien (90) in Abhängigkeit von der Wechselwirkung der elektromagnetischen Strahlung in dem betreffenden Teilbereich des elektromagnetischen Spektrums mit den Fremdmaterialien (90) zuzuordnen, die detektierten Fremdmaterialien (90) je nach ihrer Wechselwirkung mit der elektromagnetischen Strahlung in dem betreffenden Teilbereich des elektromagnetischen Spektrums in den mindestens zwei Farbklassen (411-414) zu klassieren,
bei Vorliegen einer Stichprobe mit einer Vielzahl von klassierten Fremdmaterialien (90) eine Häufigkeitsverteilung (511-514) der Fremdmaterialien für die Farbklassen zu ermitteln,
die ermittelte Häufigkeitsverteilung (511-514) mit einer Referenz-Häufigkeitsverteilung zu vergleichen und
bei einer Abweichung der ermittelten Häufigkeitsverteilung (511-514) von der Referenz-Häufigkeitsverteilung mindestens eine aus einer Menge von mehreren Optimierungshandlungen auszulösen.

13. Vorrichtung (100) nach Anspruch 12, wobei die Menge von mehreren Optimierungshandlungen mindestens eines der folgenden Elemente beinhaltet:
• ein Warnsignal wird ausgegeben;
• eine Empfehlung wird ausgegeben;
• die ermittelte Häufigkeitsverteilung (511-514) wird ausgegeben.

14. Vorrichtung (100) nach Anspruch 12 oder 13, zusätzlich beinhaltend eine Ausscheideeinheit (109) zur selektiven Ausscheidung von Objekten aus dem Textilfasergebilde (9), wobei die Auswerteeinheit (107) mit der Ausscheideeinheit (109) verbunden und dazu eingerichtet ist,
ein Ausscheidungskriterium für jede der mindestens zwei Farbklassen (411-414) zu speichern,
eine Ausscheidung der Fremdmaterialien (90) aus dem Textilfasergebilde (9) durch die Ausscheideeinheit (109) gemäss den mindestens zwei Ausscheidungskriterien auszulösen und
die Häufigkeitsverteilung (511-514) derart zu ermitteln, dass sie sich auf die aus dem Textilfasergebilde (9) ausgeschiedenen und/oder die in dem Textilfasergebilde (9) verbliebenen Fremdmaterialien (90) bezieht.

15. Vorrichtung (100) nach Anspruch 14, wobei die Menge von mehreren Optimierungshandlungen mindestens eines der folgenden Elemente beinhaltet:
• das für die betreffende Farbklasse (411-414) definierte Ausscheidungskriterium wird so geändert, dass bei unverändertem Textilfasergebilde (9) in der betreffenden Farbklasse (411-414) mehr Fremdmaterialien (90) aus dem Textilfasergebilde (9) ausgeschieden werden;
• das für die betreffende Farbklasse (411-414) definierte Ausscheidungskriterium wird so geändert, dass bei unverändertem Textilfasergebilde (9) in der betreffenden Farbklasse (411-414) weniger Fremdmaterialien (90) aus dem Textilfasergebilde (9) ausgeschieden werden.

## Claims

1. Method for optimizing a yarn production process in which foreign materials (90) are monitored in a textile fiber formation (9), e.g., a fiber flock stream or a yarn, wherein the textile fiber formation (9) is irradiated with electromagnetic radiation from at least two different subranges of the electromagnetic spectrum,
the electromagnetic radiation interacts with the foreign materials (90), and
foreign materials (90) are detected based on their interaction with the electromagnetic radiation,
**characterized in that**
a color class (411-414) of foreign materials (90) is assigned to each of at least two different subranges of the electromagnetic spectrum depending on the interaction of the electromagnetic radiation in the respective subrange of the electromagnetic spectrum with the foreign materials (90),
the detected foreign materials (90) are automatically classified in the at least two color classes (411-414) depending on their interaction with the electromagnetic radiation in the relevant subrange of the electromagnetic spectrum,
when a sample having a plurality of classified foreign materials (90) is available, a frequency distribution (511-514) of the foreign materials (90) is automatically determined for the color classes (411-414),
the determined frequency distribution (511-514) is automatically compared with a reference frequency distribution, and
in case of a deviation of the determined frequency distribution (511-514) from the reference frequency distribution, at least one of a set of several optimization actions is performed automatically.

2. Method according to claim 1, wherein the set of multiple optimization actions includes at least one of the following:
• a warning signal is output;
• a recommendation is issued;
• the determined frequency distribution (511-514) is output.

3. Method according to claim 1 or 2, wherein
a separation criterion is defined for each of the at least two color classes (411-414), foreign materials (90) are separated from the textile fiber formation (9) according to the at least two separation criteria, and
the frequency distribution relates to the foreign materials (90) separated from the textile fiber formation (9) and/or the foreign materials (90) remaining in the textile fiber formation (9).

4. Method according to claim 3, wherein the set of multiple optimization actions includes any of the following:
• the separation criterion defined for the relevant color class (411-414) is changed in such a way that, with the textile fiber formation (9) remaining unchanged, more foreign materials (90) in the relevant color class (411-414) are separated from the textile fiber formation (9);
• the separation criterion defined for the relevant color class (411-414) is changed in such a way that, with the textile fiber formation (9) remaining unchanged, fewer foreign materials (90) in the relevant color class (411-414) are separated from the textile fiber formation (9).

5. Method according to claim 3 or 4, wherein the at least two separation criteria are dependent on a reflectivity and/or transmissivity of the foreign materials (90) in the relevant subrange of the electromagnetic spectrum.

6. Method according to one of the claims 3-5, wherein the at least two separation criteria are dependent on a spatial extension of the foreign materials (90).

7. Method according to one of the preceding claims, wherein the reference frequency distribution is dependent on at least one previously determined frequency distribution (511-514) of the foreign materials (90) for the color classes (411-414).

8. Method according to one of the claims 1-6, wherein the reference frequency distribution is dependent on at least one frequency distribution of the foreign materials for the color classes (411-414) determined on another, similar textile fiber formation.

9. Method according to one of the claims 1-6, wherein the reference frequency distribution is dependent on at least one frequency distribution of the foreign materials for the color classes (411-414) determined at a textile fiber formation produced from the textile fiber formation (9) downstream in the textile production process, and preferably the textile fiber formation (9) is a fiber flock stream and the downstream textile fiber formation is a yarn.

10. Method according to one of the claims 1-6, wherein the reference frequency distribution is dependent on at least one frequency distribution of the foreign materials for the color classes (411-414) determined at a textile fiber formation produced from the textile fiber formation (9) upstream in the textile production process, and preferably the textile fiber formation (9) is a yarn and the upstream textile fiber formation is a fiber flock stream.

11. Method according to one of the preceding claims, wherein
during the interaction of the electromagnetic radiation with the foreign materials (90), an image (300) or one image each of the textile fiber image (9) is recorded in the at least two different subranges of the electromagnetic spectrum,
the image (300) or the at least two images are processed by means of image processing, and
the foreign materials (90) are detected based on data resulting from the corresponding image processing.

12. Device (100), e.g., a fiber cleaner or a yarn clearer, for optimizing a yarn production process in which foreign materials (90) are monitored in a textile fiber formation (9), including
at least one light source (103) for illuminating the textile fiber formation (9) with electromagnetic radiation from at least two different subranges of the electromagnetic spectrum,
at least one sensor system (105) for detecting foreign materials (90) based on their interaction with the electromagnetic radiation, and
an evaluation unit (107) connected to the sensor system (105) for evaluating an output signal of the sensor system (105),
**characterized in that**
the evaluation unit (107) is configured
to assign a color class (411-414) of foreign materials (90) to each of at least two different subranges of the electromagnetic spectrum depending on the interaction of the electromagnetic radiation in the respective subrange of the electromagnetic spectrum with the foreign materials (90),
to classify the detected foreign materials (90) in the at least two color classes (411-414) depending on their interaction with the electromagnetic radiation in the relevant subrange of the electromagnetic spectrum,
when a sample having a plurality of classified foreign materials (90) is available, to determine a frequency distribution (511-514) of the foreign materials for the color classes,
to compare the determined frequency distribution (511-514) with a reference frequency distribution, and
to trigger at least one of a set of several optimization actions in case of a deviation of the determined frequency distribution (511-514) from the reference frequency distribution.

13. Device (100) according to claim 12, wherein the set of multiple optimization actions includes at least one of the following:
• a warning signal is output;
• a recommendation is issued;
• the determined frequency distribution (511-514) is output.

14. Device (100) according to claim 12 or 13, additionally containing a separation unit (109) for selectively separating objects from the textile fiber formation (9), wherein the evaluation unit (107) is connected to the separation unit (109) and is configured
to store a separation criterion for each of at least two color classes (411-414), to trigger a separation of the foreign materials (90) from the textile fiber formation (9) by the separation unit (109) according to the at least two separation criteria, and
to determine the frequency distribution (511-514) in such a way that it relates to the foreign materials (90) separated from the textile fiber formation (9) and/or the foreign materials (90) remaining in the textile fiber formation (9).

15. Device (100) according to claim 14, wherein the set of multiple optimization actions includes at least one of the following:
• the separation criterion defined for the relevant color class (411-414) is changed in such a way that, with the textile fiber formation (9) remaining unchanged, more foreign materials (90) in the relevant color class (411-414) are separated from the textile fiber formation (9);
• the separation criterion defined for the relevant color class (411-414) is changed in such a way that, with the textile fiber formation (9) remaining unchanged, fewer foreign materials (90) in the relevant color class (411-414) are separated from the textile fiber formation (9).

## Revendications

1. Procédé pour optimiser un processus de fabrication de fil, dans lequel la présence de matières étrangères (90) dans une structure de fibres textiles (9), par exemple un flux de flocons de fibres ou un fil, est surveillée, dans lequel
la structure de fibres textiles (9) est exposée à un rayonnement électromagnétique dans au moins deux bandes différentes du spectre électromagnétique,
le rayonnement électromagnétique interagit avec les matières étrangères (90) et
les matières étrangères (90) sont détectées en raison de leur interaction avec le rayonnement électromagnétique,
**caractérisé en ce que**
chacune des au moins deux bandes différentes du spectre électromagnétique est associée à une classe de couleur (411-414) de matières étrangères (90) selon l'interaction du rayonnement électromagnétique de la bande en question du spectre électromagnétique avec les matières étrangères (90) ;
les matières étrangères (90) détectées sont automatiquement classées, selon leur interaction avec le rayonnement électromagnétique dans la bande en question du spectre électromagnétique, dans les au moins deux classes de couleur (411-414) ; quand un échantillon contenant plusieurs matières étrangères (90) se présente, une distribution de fréquence (511-514) des matières étrangères (90) est automatiquement déterminée pour les classes de couleur (411-414) ;
la distribution de fréquence déterminée (511-514) est comparée automatiquement à une distribution de fréquence de référence et
en cas d'écart entre la distribution de fréquence déterminée (511-514) et la distribution de fréquence de référence, au moins une action parmi un ensemble de plusieurs actions d'optimisation est exécutée automatiquement.

2. Procédé selon la revendication 1, dans lequel l'ensemble de plusieurs actions d'optimisation comprend au moins un des éléments suivants :
• émission d'un signal d'avertissement ;
• émission d'une recommandation ;
• émission de la distribution de fréquence déterminée (511-514).

3. Procédé selon la revendication 1 ou 2, dans lequel
un critère d'élimination est défini pour chacune des au moins deux classes de couleur (411-414) ;
les matières étrangères (90) sont éliminées de la structure de fibres textiles (9) selon les au moins deux critères d'élimination et
la distribution de fréquence fait référence aux matières étrangères (90) éliminées de la structure de fibres textiles (9) et/ou restées dans la structure de fibres textiles (9).

4. Procédé selon la revendication 3, dans lequel l'ensemble de plusieurs actions d'optimisation comprend l'un des éléments suivants :
• modification du critère d'élimination défini pour la classe de couleur (411-414) en question de telle sorte que, sans changement de la structure de fibres textiles (9) dans la classe de couleur (411-414) en question, davantage de matières étrangères (90) soient éliminées de la structure de fibres textiles (9) ;
• modification du critère d'élimination défini pour la classe de couleur (411-414) en question de telle sorte que, sans changement de la structure de fibres textiles (9) dans la classe de couleur (411-414) en question, moins de matières étrangères (90) soient éliminées de la structure de fibres textiles (9).

5. Procédé selon la revendication 3 ou 4, dans lequel les au moins deux critères d'élimination dépendent d'une réflectivité et/ou d'une transmissivité des matières étrangères (90) dans la bande en question du spectre électromagnétique.

6. Procédé selon l'une des revendications 3-5, dans lequel les au moins deux critères d'élimination dépendent d'une extension spatiale des matières étrangères (90).

7. Procédé selon l'une des revendications précédentes, dans lequel la distribution de fréquence de référence dépend d'au moins une distribution de fréquence (511-514) des matières étrangères (90) pour les classes de couleur (411-414) déterminée au préalable.

8. Procédé selon l'une des revendications 1 à 6, dans lequel la distribution de fréquence de référence dépend d'au moins une distribution de fréquence des matières étrangères déterminée pour les classes de couleur (411-414) dans au moins une autre structure de fibres textiles de même nature.

9. Procédé selon l'une des revendications 1 à 6, dans lequel la distribution de fréquence de référence dépend d'au moins une distribution de fréquence des matières étrangères pour les classes de couleur (411-414) déterminée dans au moins une structure de fibres textiles fabriquée à partir de la structure de fibres textiles (9) dans la suite du processus de fabrication textile et la structure de fibres textiles (9) est de préférence un flux de flocons de fibres et la structure de fibres textiles suivante est un fil.

10. Procédé selon l'une des revendications 1 à 6, dans lequel la distribution de fréquence de référence dépend d'au moins une distribution de fréquence déterminée des matières étrangères pour les classes de couleur (411-414) déterminée sur au moins une structure de fibres textiles antérieure dans le processus de fabrication textile, à partir de laquelle la structure de fibres textiles (9) est fabriquée, et la structure de fibres textiles (9) est de préférence un fil et la structure de fibres textiles antérieure est un flux de flocons de fibres.

11. Procédé selon l'une des revendications précédentes dans lequel,
pendant l'interaction du rayonnement électromagnétique avec les matières étrangères (90), une image (300) ou une image respective de la structure de fibres textiles (9) est acquise dans les au moins deux bandes différentes du spectre électromagnétique,
l'image (300) ou les au moins deux images sont traitées au moyen d'un traitement d'image et
les matières étrangères (90) sont détectées sur la base des données issues du traitement d'image correspondant.

12. Dispositif (100), par exemple nettoyeur de fibres ou nettoyeur de fil, pour optimiser un processus de fabrication de fil, dans lequel des matières étrangères (90) sont surveillées dans une structure de fibres textiles (9), comprenant
au moins une source lumineuse (103) pour exposer la structure de fibres textiles (9) à un rayonnement électromagnétique dans au moins deux bandes du spectre électromagnétique,
au moins un système de capteurs (105) pour la détection de matières étrangères (90) par leur interaction avec le rayonnement électromagnétique et
une unité d'analyse (107) connectée au système de capteurs (105) pour l'analyse d'un signal de sortie du système de capteurs (105),
**caractérisé en ce que**
l'unité d'analyse (107) est configurée pour
associer chacune des au moins deux bandes différentes du spectre électromagnétique à une classe de couleur (411-414) de matières étrangères (90) en fonction de l'interaction du rayonnement électromagnétique dans la bande en question du spectre électromagnétique avec les matières étrangères (90), classer les matières étrangères (90) détectées dans les au moins deux classes de couleur (411-414) en fonction de leur interaction avec le rayonnement électromagnétique dans la bande en question du spectre électromagnétique, quand un échantillon contient plusieurs matières étrangères (90) classées, déterminer une distribution de fréquence (511-514) des matières étrangères pour les classes de couleur,
comparer la distribution de fréquence déterminée (511-514) avec une distribution de fréquence de référence et
en cas d'écart de la distribution de fréquence déterminée (511-514) par rapport à la distribution de fréquence de référence, déclencher au moins une action parmi un ensemble de plusieurs actions d'optimisation.

13. Dispositif (100) selon la revendication 12, dans lequel l'ensemble de plusieurs actions d'optimisation comprend au moins un des éléments suivants :
• émission d'un signal d'avertissement ;
• émission d'une recommandation ;
• émission de la distribution de fréquence déterminée (511-514).

14. Dispositif (100) selon la revendication 12 ou 13, comprenant en outre une unité d'élimination (109) pour l'élimination sélective d'objets de la structure de fibres textiles (9), l'unité d'analyse (107) étant connectée à l'unité d'élimination (109) et configurée pour
enregistrer un critère d'élimination pour chacune des au moins deux classes de couleur (411-414),
déclencher l'élimination des matières étrangères (90) de la structure de fibres textiles (9) par l'unité d'élimination (109) selon les au moins deux critères d'élimination et
déterminer la distribution de fréquence (511-514) de telle manière qu'elle fait référence aux matières étrangères (90) éliminées de la structure de fibres textiles (9) et/ou restées dans la structure de fibres textiles (9).

15. Dispositif (100) selon la revendication 14, dans lequel l'ensemble de plusieurs actions d'optimisation comprend au moins un des éléments suivants :
• modification du critère d'élimination défini pour la classe de couleur (411-414) en question de telle sorte que, sans changement de la structure de fibres textiles (9) dans la classe de couleur (411-414) en question, davantage de matières étrangères (90) soient éliminées de la structure de fibres textiles (9) ;
• modification du critère d'élimination défini pour la classe de couleur (411-414) en question de telle sorte que, sans changement de la structure de fibres textiles (9) dans la classe de couleur (411-414) en question, moins de matières étrangères (90) soient éliminées de la structure de fibres textiles (9).
